# EUROPEAN PATENT APPLICATION

(11) **EP 1 081 227 A1**
(43) Date of publication of application: **07.03.2001**
(21) Application number: 99202859.7
(22) Date of filing: 02.09.1999
(51) Int. Cl.: C12N 15/86, A61K 48/00

(54) **Methods and means for retroviral gene delivery**

(71) Applicant: Erasmus Universiteit Rotterdam, 3015 GE Rotterdam (NL)
(72) Inventor: The designation of the inventor has not yet been filed
(74) Representative: Ottevangers, Sietse Ulbe

(57) **Abstract**

The invention provides methods, compositions and uses of said compositions for transducing target cells with gene delivery vehicles of retroviral origin comprising providing a selection of CD34 positive cells from a culture of cells, taking a plurality of samples of said Cd34 positive population, diluting and/or concentrating said samples to provide a range of concentrations of cells per volume, contacting samples in said range with a composition comprising said gene delivery vehicles and determining the optimal concentration of target cells for efficient transduction and diluting or concentrating said CD34 positive population to said optimal concentration and contacting said population with said gene delivery vehicles to allow for said transduction of target cells.

## Description

The present invention relates to the field of recombinant retroviral particles, to retroviral gene delivery vehicles. To methods for producing particles and vehicles as well as uses of the particles or vehicles in improved transduction methods, compositions for transduction as well as pharmaceutical compositions for the treatment of disorders with a genetic component. In particular the invention provides in one of its embodiments a method of gene transfer into e.g. pluripotent hematopoietic stem cells and their descendants, enabling successful transduction of 90% of CD34+ cells, including transplantable cell populations comprising hematopoietic stem cells that give rise to progeny expressing the transduced gene(s). The use of the method e.g. includes and is included in a method for treatment of a variety of hereditary and acquired human diseases by transfer of therapeutically active genes into hematopoietic stem cells and genetic marking of such cells.

The hematopoietic system produces perpetually large numbers of blood cells, which have a limited life span and need to be perpetually renewed throughout the life of a mammal. This renewal is maintained through proliferation and differentiation of a small number of hematopoietic stem cells in the bone marrow. The definition of stem cells is not always clear within the art. Herein a functional definition is used, which defines stem cells as those cells capable of (lon term) reconstitution of a hematopoietic system. This definition is often felt to include at least some early progenitor cells. Since blood cells virtually reach every organ, hematopoietic stem cells are a highly suitable target for gene therapy for a variety of hereditary and acquired diseases within and outside the hematopoietic system. Unfortunately, retrovirus mediated gene transfer has met with only limited success due to the difficulty of obtaining sufficient numbers of successfully transduced, transplantable, long-term repopulating hematopoietic stem cells.

Recent advances in understanding stem cell biology include the discovery of heterogeneity of stem cells both in terms of maturity as well as the discovery of novel growth factors thought to control their proliferation and differentiation and the possibility to purify hematopoietic stem cells by selection for the CD34 surface marker. Retrovirus mediated gene transfer has been greatly benefited by co-localization of cells and virus using a recombinant fibronectin fragment, whereas the importance of selecting a suitable retrovirus receptor has been recognized. Based on these advances, we have analyzed the variables influencing gene transfer during the transduction procedure and selected a highly efficient producer cell subclone. With our invention we have made the observation that several binding steps involving receptors and ligands on virus, cells and mediating adherence molecules are needed for successful transduction, each with their specific affinities. Hence, our invention discloses the finding that the transduction procedure should be highly dependent on the relative concentration of these molecules during the transduction procedure.

Thus the invention provides a method for transducing target cells with gene delivery vehicles of retroviral origin comprising providing a selection of CD34 positive cells from a culture of cells, taking a plurality of samples of said Cd34 positive population, diluting and/or concentrating said samples to provide a range of concentrations of cells per volume, contacting samples in said range with a composition comprising said gene delivery vehicles and determining the optimal concentration of target cells for efficient transduction and diluting or concentrating said CD34 positive population to said optimal concentration and contacting said population with said gene delivery vehicles to allow for said transduction of target cells.

As stated herein before the population of CD34 positive cells such as they can be found in e.g. umbilical cord blood or bone marrow includes the stem cells as defined herein above (i.e. the cells capable of long term repopulation). We have found that a very important variable in the efficiency of transduction is a ratio between number of cells (or cell concentration) and the number of transducing particles. According to our invention these should be optimised vis a vis one another, which may sometimes lead to increasing particle titers and/or target cell concentrations, but surprisingly also to decreasing viral particles and/or lowering cell concentrations. Therefor a range of concentrations should be tried with samples from the target population of cells. Gene delivery vehicles of retroviral origin are all vehicles comprising genetic material and/or proteinaceous material derived from retroviruses. Typically the most important features of such vehicles are the integration of their genetic material into the genome of a target cells and their capability to transduce stem cells. These elements are deemed essential in a functional manner, meaning that the sequences need not be identical to retroviral sequences as long as the essential functions are present. The methods of the invention are however especially sutable for recombinant retroviral particles, which have most if not all of the repication and reproduction features of a retrovirus, typically in combination with a producer cell having some complementing elements. Normally the retroviral particles making up the gene delivery vehicle are recplication defective on their own. The invention is particularly suited for the production of gene delivery vehicles, however other retroviral particles can also be produced according to the invention.

In another embodiment according to the invention not only the concentration of target cells is optimised, but also the concentration of virus. As stated before optimisation of all concentrations involved in binding or interaction is preferred. In order to be able to modify virus titers high initial titers are preferred. Methods to arrive at those are also provided by the present invention. Thus the invention further provides a method wherein target cell concentrations are optimised further comprising optimising the concentration of said gene delivery vehicles for optimal transduction efficiency. It is of course that a gene delivery vehicle is intended to read on any vehicle capable of delivering genetic material to a target cell, whether the genetic material is actually a gene, an antisense molecule or a cosuppressive nucleic acid (encoding molecule), etc. Useful nucleic acids to be provided to target cells, e.g. stem cells are well known in the art and include such molecules as to replace inborn errors/deficiencies of the hematopoietic system, which may include hemoglobin genes and their regulatory elements for the thalassemia's and sickle cell anemia's and sequences to repair the various forms of severe combined immunodeficiency, such as caused by adenosine deaminase deficiency and that known as severe X linked immunodeficiency, or genes encoding enzymes for diseases known as lysosomal storage diseases, such as Hurler's, Hunter's, Krabbe's and in particular Gaucher's disease and metachromatic leukodystrophy, or by introducing sequences that confer resistance of the progeny of hematopoietic stem cells to infectious agents, such as HIV, as well as the introduction of suicide genes for cancer therapy and marker genes to track the progeny of transplanted normal and/or malignant hematopoietic stem cells. Another factor involved in binding and/or interaction is a matrix binding both virus and target cell exemplified herein by fibronectin and retronectin. The optimisation of their concentration is also part of the present invention. Thus the invention also provides a method as described above wherein said target cells are cultured in the presence of fibronectin, retronectin or a functional equivalent thereof, preferably further comprising optimising the concentration of said fibronectin, retronectin or said functional equivalent for optimal transduction efficiency. Typically the target cells of the present invention comprise poulations of CD34 positive cells, which are efficiently transduced by retroviral particles, preferred are thopse populations wherein said CD34 positive cells comprise umbilical cord blood cells or bone marrow cells. As stated above it is preferred to use high viral titers to be able to optimise all relevant concentrations vis a vis all binding and/or interaction steps. Therefor the invention also provides a method as described above wherein a composition of retroviral gene delivery vehicles of improved titer is applied. According to the invention preferably a method for improving the virus titer is applied which involves improving the producer cell line. Thus the invention also provides a method wherein said virus titer is improved by providing a culture of producer cells of a retroviral gene delivery vehicle, subcloning said culture of producer cells, culturing the resulting subclones and selects the clones producing the highest virus titers, possibly based on multiple copies of the provirus due to reinfection. Apparently a number of cells from established producer cell lines loose some of their ability to produce effective retroviral particles. Subcloning appears to be a way to select for those cells retaining that ability. Other ways of selecting for such cells are also included in the present invention.

The method of improving virus titers can also be use apart from the improvement of transduction. Thus the invention also provides a method for producing retroviral particles at high titers, comprising providing a culture of producer cells producing retroviral particles, subcloning said culture of producer cells, culturing the resulting subclones and selecting the cultures producing the highest virus titers. Again the invention is preferably applied to gene delivery vehicle produciton. Thus the invention provides in yet another embodiment a method as just described wherein said retroviral particles are gene delivery vehicles. Typically producer cells are well known in the art. The preferred ones are mouse fibroblast cells, originating from PG13 which is pseudotyped with the gibbon ape leukemia virus (GALV). GALV-receptors (GLV-1 or Pit-1) are present on human hematopoietic cells. The invention also includes compositions obtainable by the methods of the invention. Thus included are compositions comprising retroviral particles at high titer obtainable by a method as disclosed above, preferably those wherein said retroviral particles are gene delivery vehicles. Preferably such a composition comprises retroviral particles capable of transducing hematopoietic stem cells and/or progenitor cells, preferably wherein said retroviral particles are capable of transducing umbilical cord blood cells and/or bone marrow cells.

The invention also provides the pharmaceutical use of these compositions, particularly in the treatment of diseases having a genetic component, such as the various genetic hemoglobin orders, the large group of rare diseases collectively know as severe combined immune deficiencies, the group of lysosomal storage diseases, especially with a strong hematopoietic and/or visceral expression, such as Gaucher's disease, but also possibly Krabbe's disease, as well as in the treatment of infectious disease, notably HIV infection, or cancer, but also in the treatment of infectious disease or cancer. Typically the use of a composition comprising retroviral particles will involve the transduction of CD34 positive target cells. Such transduced cells are typically made in vitro and are also part of the present invention. Thus the invention provides a composition for the treatment of a hereditary disease or a pathological condition related to a genetic defect or a genetic aberration, comprising a plurality of CD34 positive cells transduced with a composition of retroviral particles according to the invention, or a composition for the treatment of a hereditary disease or a pathological condition related to a genetic defect or a genetic aberration, comprising a plurality of CD34 positive cells, said composition being obtainable by a method according to the invention.

### Detailed description.

The object of the present invention is to provide a generally applicable method for retrovirus mediated transfer of therapeutic and marker genes into pluripotent hematopoietic stem cells.

The invention includes the unexpected and surprising the preferred ones are mouse fibroblast cells, originating from PG13 which is pseudotyped with the gibbon ape leukemia virus (GALV). GALV-receptors (GLVR-1 or Pit-1) are present on human hematopoietic cells.
dependent on the concentration of target cells during the transduction period and that selection of (high titer) subclones of a by itself effective producer cell line decreases this dependence on cell concentration. These two findings enable a reproducible, highly efficient method of gene transfer into hematopoietic stem cells, which maintain their transplantability and provide descendant containing the gene of interest and expressing this gene following transplantation in recipient subjects. Proof of principle will be obtained using transplantation of successfully marked CD34+ cells into irradiated nonhuman primates. For human umbilical cord blood stem cells, provisional proof of principle has already been obtained (Van Hennik et al., BLOOD, 1998) by transplantation of transduced stem cells into immunodeficient NOD/SCID recipients under conditions resulting in a lower transduction frequency of CD34+ than has become possible by the present invention. Hence, clinical application of the method for autologous umbilical cord blood gene therapy for a variety of diseases has become a feasible option for therapy by the present invention.

### Materials and Methods

### Viral vector and packaging cell line

The pseudotyped retroviral producer cell line PG13/EGFP7 was kindly provided by J. Barquinero (Institut de Recerca Oncologica, Barcelona, Spain). The cell line was developed by transducing the PG13 packaging cell line (kindly provided by D. Miller, Fred Hutchinson Cancer Research Center, Seattle, WA) with 0.45 µm filtered supernatant from PA317/EGFP cell cultures. (Limon A et al., (1997), Blood, 90:3316-21 21). EGFP expression was analyzed by flow cytometry and bright single cells were sorted on 96-well plates by using an EPICS Elite ESP flow cytometer coupled to an autoclone device (both from Coulter, Miami, FL, USA). Single clones were cultured as previously described. (Limon A et al., (1997), Blood, 90: 3316-21). The PG13/EGFP7 cell line was subcloned by diluting the cells to 1 cell per well of a 96-well plate. Single subclones were cultured as described and analyzed for transduction efficiency on rhesus BM and UCB CD34⁺ cells. The viral titer of the cell line (original and subclones) was in the order of 10⁵ - 10⁶ infectious particles per ml as determined by supernantant titration on cultured human HeLa cells and Rat-2 cells. Absence of replication-competent virus was verified by failure to transfer GFP-expression from a transduced cell population to a secondary population.

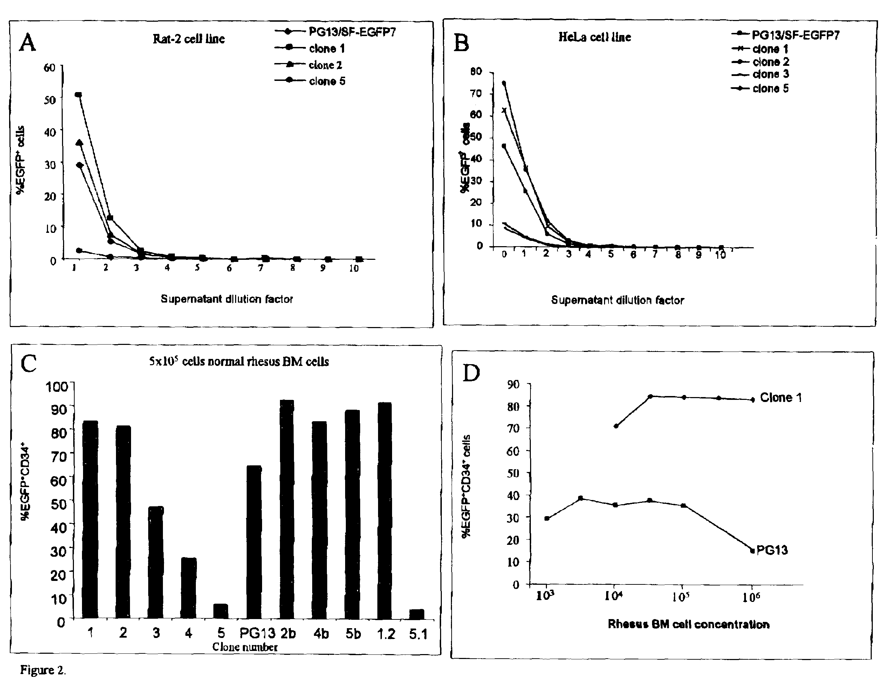

### Subcloning of the PG13/SF-EGFP packaging cell line/vector combination (Figure 2).

Subcloning of the PG13/SF-EGFP7 virus producer cell line was performed using limiting dilution to grow one cell per well of a 96-well plate in culture medium consisting of an enriched version of Dulbecco's modified Eagle's medium (DMEM, Gibco, Gaithersburg, MD). (Merchav S et al. 1984), Int J Cell Cloning, 2: 356-67). (Wagemaker G et al. (1980), Cell Tissue Kinet, 13: 505-17). Supplemented with 10% FCS. Growing clones were harvested and grown in T75cm² flasks until 80% confluency and subsequently tested for transduction efficiency.

### Determination of the virus titer (Figure 2 and Table 1)

To determine the virus titer of all clones, diluted supernatants of these clones were used to transduce cells of the Rat-2 cell line and HeLa cell line. The producer cell lines (PG13/SF-EGFP7, clone 1, clone 2, clone 3 and clone 5) were grown in T75 cm² culture flasks untill 80% confluency as described above. Subsequently, 2000 Rat-2 cells and HeLa cells were cultured for 24 hours in dilutions of 0.45 µm filtered virus supernatant of the different clones of the virus producer cell line in 12 wells of a 24-wells plate. As a control, 1 well did not contain virus supernatant but culture medium solely. The virus supernatant was removed and substituted with fresh culture medium. The transduced cells were harvested at confluency and the transduction efficiency was determined by flow cytometry (FACSCalibur, Becton&Dickinson). The virus titer was determined by calculating the number of cells initially cultured (2000) that were transduced at a certain dilution of the virus supernatant.

### Retroviral transduction

Supernatants containing recombinant retrovirus were generated by culturing approximately 80% confluent producer cells for 12 hours in culture medium consisting of serum-free enriched version of Dulbecco's modified Eagle's medium (DMEM, Gibco, Gaithersburg, MD). (Merchav S et al. (1984), Int J Cell Cloning, 2: 356-67). (Wagemaker et al. (1980), Cell Tissue Kinet, 13: 505-17). Media for all cultures routinely included 100 U/ml of penicillin and 100 µg/ml of streptomycin. The cultures were maintained at 37°C with 10% CO₂ (measured every 15' with read-outs between 9.5% and 10%) in a humidified atmosphere. The culture supernatant was subsequently produced and passed through a 0.45 µm filter. To enhance the transduction efficiency, Falcon 1008 (35 mm) bacteriological culture dishes were coated with the recombinant fibronectin fragment CH-296 (Takara Shuzo, Otsu, Japan) at a concentration of 10 µg/cm² as described previously. (Moritz T et al. (1996), Blood, 88: 855-62). CD34-selected UCB, human BM, normal rhBM or rhBM from G-CSF and/or Flt3-L treated monkeys were prestimulated for 2 days in enriched Dulbecco's medium with combinations of the human recombinant growth factors Flt3-L (50 ng/ml, kindly provided by Amgen, Thousand Oaks, CA, USA), thrombopoietin (huTPO and rhTPO, 10 ng/ml, kindly provided by Genentech, South San Francisco, CA, USA) and stem cell factor (SCF, 100 ng/ml). Before adding purified subsets to the fibronectin-coated dishes, the CH-296 fibronectin fragment was preincubated with supernatant containing the pseudotyped vector for 1 hour at 37°C. (Moritz et al.(1996), Blood, 88: 855-62). (Hanenberg H et al. (1996), Nat Med, 2: 876-82). Subsequently, nucleated cells were resuspended in the vector-containing supernatant supplemented with hematopoietic growth factors (HGF), and added to the dishes. Over a period of 2 days, culture supernatant was replaced completely by resuspending nonadherent cells into fresh retrovirus supernatant and HGF. Finally, the cells were harvested and analysed by flow cytometry and cell cycle analysis.

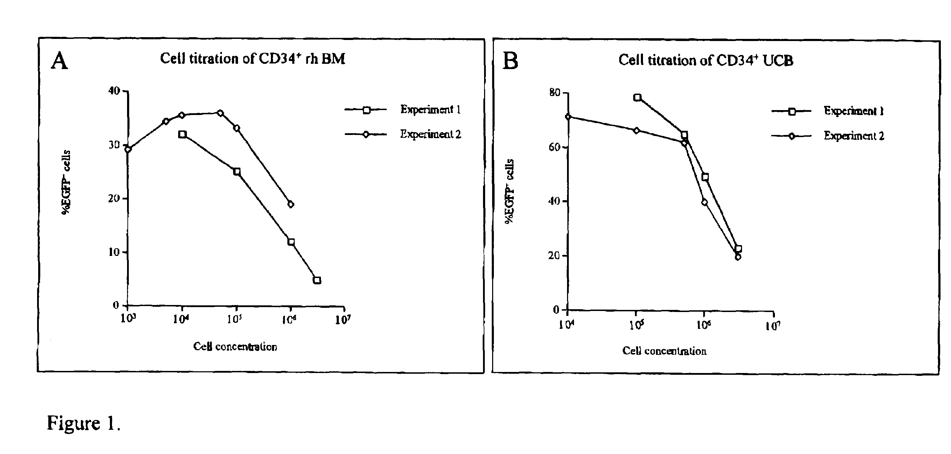

### Target cell titrations (Figure 1)

Rhesus monkey BM (rh BM) and human umbilical cord blood (UCB) cells were titrated from 3x10⁶/ml to 10³/ml during the transduction assay. The producer cell line was, as standard, cultured in T75 cm² flasks filled with 10 ml serumfree medium as described above untill 80% confluency. During the transduction, the virus supernatant was refreshed once. After 2 days prestimulation and 2 days of supernatant infection the cells were harvested and the transduction efficiency was determined by flow cytometry.

### Transplantation assays in rhesus monkeys

To test for transduction of transplantable multilineage and/or pluripotent hematopoietic stem cells, EGFP-transduced CD34+ cells are transplanted into rhesus monkeys subjected to 9 Gy (6 MV X-rays) total body irradiation in cell numbers range from hundred thousand to several millions of CD34+ cells per kg body weight, and either immediately following transduction or selected for expression of the the EGFP gene by cell sorting using a FACS Vantage cell sorter (Becton Dickinson). The transplantation procedure has been described in detail (Neelis KJ et al. (1997), Exp Hematol, 25: 1094-103). The monkeys are followed daily for expression of EGFP in peripheral blood subsets, and weekly for expression in bone marrow subsets, using flow cytometric surface marker labeling to identify the different blood cell lineages.

### Brief Description of the Drawings

### Background Figure

The structure of the α-chain of fibronectin and the human recombinant fibronectin fragment (retronectin), CH-296, and the domains involved in colocalization of target cells and retrovirus are schematically represented. The ligands by which cell binding takes place are VLA-5 in the cell binding domain (CBD), VLA-4 in the CS1 peptide and the proteoglycans in the heparin domain.

### Figure 1.

Cell titration of CD34-selected rhesus BM cells (A) and human umbilical cord blood (UCB) (B) during infection with the GALV-pseudotyped PG13/SF-EGFP7 retroviral packaging cell line/vector combination. The highest levels of EGFP⁺ cells were found after transduction of 5x10⁴/ml rh BM cells or 10⁵/ml UCB cells with 35% and 80%, respectively.

### Figure 2.

Subcloning of the PG13/SF-EGFP7 packaging cell line/vector combination resulted in clones with different virus titers as determined by supernatant dilution on Rat-2 cells (A) and HaLa cells (B). Clone 1 and 2 (PG13/SF-EGFP7.1 and PG13/SF-EGFP7.2) showed the highest virus titers, whereas clone 3 and 5 (PG13/SF-EGFP7.3 and PG13/SF-EGFP7.5) resulted in low virus titers. Transduction of 5x105 rh BM cells with PG13/SF-EGFP7 and clones 1 - 5, 2b, 4b, and 5b (C) resulted in levels of EGFP-transduced rh BM cells that correlates with the virus titers. Clone 1, 2 and 2b showed EGFP levels of 80%-90% which was higher as compared to the parental PG13/SF-EGFP7 producer. Subclones of clone 1 and 5 (PG13/SF-EGFP7.1.2 and PG13/SF-EGFP7.5.1, respectively) resulted in similar transduction levels as the parental cell lines. Decreasing cell numbers (from 10⁶ to 10³ cells/ml) during transduction using virus supernatant of PG13/SF-EGFP7 resulted in increasing transduction efficiency ranging from 10% to 40% (D). Transduction with supernatant from subclone PG13/SF-EGFP7.1 resulted in higher efficiencies (80% to 90% EGFP⁺ cells) without the titration effect caused by increasing cell numbers/ml.

### REFERENCES

1. Limon A, Briones J, Puig T, Carmona M, Fornas 0, Cancelas JA, Nadal M, Garcia J, Rueda F and Barquinero J, (1997), High-titer retroviral vectors containing the enhanced green fluorescent protein gene for efficient expression in hematopoietic cells. Blood, 90: 3316-21.
2. Merchav S and Wagemaker G, (1984), Detection of murine bone marrow granulocyte/macrophage progenitor cells (GM-CFU) in serum-free cultures stimulated with purified M-CSF or GM-CSF. Int J Cell Cloning, 2: 356-67.
3. Wagemaker G and Visser TP, (1980), Erythropoietin-independent regeneration of erythroid progenitor cells following multiple injections of hydroxyurea. Cell Tissue Kinet, 13:505-17.
4. Moritz T, Dutt P, Xiao X, Carstanjen D, Vik T, Hanenberg H and Williams DA, (1996), Fibronectin improves transduction of reconstituting hematopoietic stem cells by retroviral vectors:evidence of direct viral binding to chymotryptic carboxy-terminal fragments. Blood, 88: 855-62.
5. Hanenberg H, Xiao XL, Dilloo D, Hashino K, Kato I and Williams DA, (1996), Colocalization of retrovirus and target cells on specific fibronectin fragments increases genetic transduction of mammalian cells. Nat Med, 2: 876-82.
6. Neelis KJ, Dubbelman YD, Wognum AW, Thomas GR, Eaton DL, Egeland T and Wagemaker G, (1997), Lack of efficacy of thrombopoietin and granulocyte colony-stimulating factor after high dose total-body irradiation and autologous stem cell or bo9ne marrow transplantation in rhesus monkeys. Exp Hematol, 25: 1094-103.
7. Hoogerbrugge PM, Suzuki K, Suzuki KU, Poorthuis BJHM, Kobayashi T, Wagemaker G, Van Bekkum DW. Donor-derived cells in the central nervous system of twitcher mice after bone marrow transplantation. Science 1988, 239: 1035 - 1038.

## Claims

1. A method for transducing target cells with gene delivery vehicles of retroviral origin comprising providing a selection of CD34 positive cells from a culture of cells, taking a plurality of samples of said Cd34 positive population, diluting and/or concentrating said samples to provide a range of concentrations of cells per volume, contacting samples in said range with a composition comprising said gene delivery vehicles and determining the optimal concentration of target cells for efficient transduction and diluting or concentrating said CD34 positive population to said optimal concentration and contacting said population with said gene delivery vehicles to allow for said transduction of target cells.

2. A method according to claim 1 further comprising optimising the concentration of said gene delivery vehicles for optimal transduction efficiency.

3. A method according to claiml or 2 wherein said target cells are cultured in the presence of fibronectin, retronectin or a functional equivalent thereof.

4. A method according to claim 3 further comprising optimising the concentration of said fibronectin, retronectin or said functional equivalent for optimal transduction efficiency.

5. A method according to any one of claims 1-4, wherein said CD34 positive cells comprise umbilical cord blood cells or bone marrow cells.

6. A method according to any one of the afore going claims wherein composition of retroviral gene delivery vehicles of improved titer is applied.

7. A method according to claim 6 wherein said virus titer is improved by providing a culture of producer cells of a retroviral gene delivery vehicle, subcloning said culture of producer cells, culturing the resulting subclones and selecting the cultures producing the highest virus titers.

8. A method for producing retroviral particles at high titers, comprising providing a culture of producer cells producing retroviral particles, subcloning said culture of producer cells, culturing the resulting subclones and selecting the cultures producing the highest virus titers.

9. A method according to claim 8, wherein said retroviral particles are gene delivery vehicles.

10. A method according to any one of claims 7-9 wherein said producer cells are cells of hematopoietic origin.

11. A method according to claim 10 wherein said producer cells originate from PG13.

12. A composition comprising retroviral particles at high titer obtainable by a method according to claim 8.

13. A composition according to claim 12 wherein said retroviral particles are gene delivery vehicles.

14. A composition according to claim 12 or 13 wherein said retroviral particles are capable of transducing hematopoietic stem cells and/or progenitor cells.

15. A composition according to any one of claims 12-14 wherein said retroviral particles are capable of transducing umbilical cord blood cells and/or bone marrow cells.

16. A composition according to any one of claims 12-15 for use as a pharmaceutical.

17. Use of a composition according to any one of claims 12-15 in the transduction of CD34 positive target cells.

18. A composition for the treatment of a hereditary disease or a pathological condition related to a genetic defect or a genetic aberration, comprising a plurality of CD34 positive cells transduced with a composition according to any one of claims 12-15.

19. A composition for the treatment of a hereditary disease or a pathological condition related to a genetic defect or a genetic aberration, comprising a plurality of CD34 positive cells, said composition being obtainable by a method according to any one of claims 1-11.

20. Use of a composition according to claim 18 or 19 in the preparation of a medicament for the treatment of the various genetic hemoglobin orders, the large group of rare diseases collectively know as severe combined immune deficiencies, the group of lysosomal storage diseases, especially with a strong hematopoietic and/or visceral expression, such as Gaucher's disease, but also possibly Krabbe's disease, as well as in the treatment of infectious disease, notably HIV infection, or cancer.
